# EUROPÄISCHE PATENTANMELDUNG

(11) **EP 4 520 280 A1**
(43) Veröffentlichungstag der Anmeldung: **12.03.2025**
(21) Anmeldenummer: 24198397.2
(22) Anmeldetag: 04.09.2024
(51) Int. Cl.: A61B 17/34

(54) **DICHTUNGSVORRICHTUNG FÜR EIN CHIRURGISCHES INSTRUMENT UND VERFAHREN**

(30) Priorität: 06.09.2023 DE 102023123938
(71) Anmelder: Karl Storz SE & Co. KG, 78532 Tuttlingen (DE)
(72) Erfinder: EGLE, Michael, 78532 Tuttlingen (DE); HERMLE, Rainer, 78532 Tuttlingen (DE); KASPARIK, Günther, 78532 Tuttlingen (DE); LÖFFLER, Oliver, 78532 Tuttlingen (DE); WITTKE, Uwe, 78532 Tuttlingen (DE)

(57) **Zusammenfassung**

Die vorliegende Erfindung betrifft eine Dichtungsvorrichtung für zumindest ein chirurgisches Instrument, mit einem Dichtelement, das zur Abdichtung einer Schnittstelle zwischen einem Arbeitselement und zumindest einem Instrument ausgebildet ist, mit einem Arretierungselement, das mit dem Arbeitselement verbindbar ist oder an diesem ausgeformt ist, wobei das Arretierungselement zur Fixierung des Dichtelements ausgebildet ist und zumindest einen Rastabschnitt aufweist, wobei das Dichtelement zum Eingreifen in den Rastabschnitt und selbsttätigen Halten an dem Arretierungselement in einer die Schnittstelle abdichtenden Position ausgebildet ist. Die vorliegende Erfindung betrifft ferner ein Verfahren zur Montage einer Dichtungsvorrichtung.

## Beschreibung

### GEBIET DER ERFINDUNG

Die vorliegende Erfindung betrifft eine Dichtungsvorrichtung für ein chirurgisches Instrument sowie ein Verfahren zur Montage einer Dichtungsvorrichtung.

### TECHNISCHER HINTERGRUND

Chirurgische Instrumente werden für unterschiedliche Anwendungen eingesetzt. Beispielsweise können diese im Bereich minimalinvasiver Operationen verwendet werden und über ein chirurgisches Werkzeug verfügen. Chirurgische Instrumente können als Resektoskop ausgebildet sein und über ein Arbeitselement verfügen, das beispielsweise zur Führung des chirurgischen Werkzeugs, wie einer Elektrode, ausgebildet ist.

Resektoskope sind insbesondere für urologische oder beispielsweise gynäkologische Anwendungen bekannt. Dabei kann das Arbeitselement mit der Elektrode durch die Harnröhre bis zu einem Operationsgebiet in den Körper eines Patienten eingeführt werden. An einem distalen Ende (d.h. benutzerfern) weist die Elektrode beispielsweise eine Schneidschlinge auf, mittels welcher Gewebe abgetragen werden kann. Das abgetragene Gewebe kann mittels einer Spülflüssigkeit, die durch einen Spülkanal bzw. Zulaufkanal zum Operationsgebiet geführt wird, durch einen Ablaufkanal wieder abgeführt werden. Der Zulauf- und der Ablaufkanal ermöglichen eine kontinuierliche Spülung, die zugleich zum Freihalten eines Sichtfensters dient, um stets eine einwandfreie endoskopische Sicht zu ermöglichen.

Chirurgische Instrumente weisen allgemein eine Handhabe mit einem Schlitten auf, der entlang einer Längsachse des chirurgischen Instruments verschiebbar gelagert und mit dem chirurgischen Werkzeug verbunden ist. Wenn ein Instrument, wie eine Elektrode, in das Arbeitselement eingesetzt ist, kann durch axiales Bewegen des Schlittens die Schneidschlinge der Elektrode in axialer Richtung verschoben werden, um beispielsweise Gewebe abzutragen.

Der Bereich, an welchem die Elektrode in das Arbeitselement eingesetzt ist, muss abgedichtet werden. Dazu sind unterschiedliche Dichtelemente bekannt.

Beispielsweise existieren zur Abdichtung Elemente aus PTFE (Polytetrafluorethylen, auch als "Teflon" bezeichnet). Derartige Teflonelemente, wie beispielsweise Teflonbuchsen, können zur Abdichtung händisch eingesetzt werden. Der Austausch derartiger Dichtelemente, beispielsweise zu Reinigungszwecken, ist jedoch zeitaufwändig. Weiterhin nachteilig kann eine Abdichtung durch derartige Teflonelemente nur sichergestellt werden, wenn ein Instrument in das Arbeitselement eingesetzt ist. Ist das Instrument nicht in das Arbeitselement eingeschoben, so resultiert eine Öffnung, die nicht abdichtet.

Aus der Druckschrift EP 2 143 393 B1 ist weiterhin eine Schutzvorrichtung für ein Dichtelement bekannt, wobei ein Deckel mit seitlichen Laschen an dem Instrumentenschaft einrasten kann. Zwei gegenüberliegende Laschen mit Rastvorsprüngen sind an dem Deckel angeordnet. Die Rastvorsprünge können in korrespondierenden Rastkanten an der Dichtelementhalterung am Instrument einrasten.

Nachteilig kann das Dichtelement nicht als komplettes Element durch die Hakenbefestigung entnommen werden, da die Befestigung nur den Deckel der Dichtung fixiert. Dadurch ist der Aufwand beim Austausch der kompletten Dichtungseinheit weiterhin hoch.

Durch die mehrfache Verwendung bekannter Dichtelemente mit Reinigungsvorgängen zwischen den Anwendungen sind die Dichtelemente allgemein aufwändig gestaltet.

### ZUSAMMENFASSUNG DER ERFINDUNG

Vor diesem Hintergrund liegt der vorliegenden Erfindung die Aufgabe zugrunde, eine verbesserte Dichtungsvorrichtung bzw. Verfahren zur Montage einer Dichtungsvorrichtung anzugeben.

Erfindungsgemäß wird diese Aufgabe durch eine Dichtungseinrichtung mit den Merkmalen des Patentanspruchs 1 und/oder durch ein Verfahren mit den Merkmalen des Patentanspruchs 12 gelöst.

Demgemäß ist vorgesehen:
Eine Dichtungsvorrichtung für zumindest ein chirurgisches Instrument, mit einem Dichtelement, das zur Abdichtung einer Schnittstelle zwischen einem Arbeitselement und zumindest einem Instrument ausgebildet ist, mit einem Arretierungselement, das mit dem Arbeitselement verbindbar ist oder an diesem ausgeformt ist, wobei das Arretierungselement zur Fixierung des Dichtelements ausgebildet ist und zumindest einen Rastabschnitt aufweist, wobei das Dichtelement zum Eingreifen in den Rastabschnitt und selbsttätigen Halten an dem Arretierungselement in einer die Schnittstelle abdichtenden Position ausgebildet ist.
Ein Verfahren zur Montage einer Dichtungsvorrichtung zur Abdichtung einer Schnittstelle zwischen einem Arbeitselement und zumindest einem Instrument eines chirurgischen Instruments, mit den Schritten: Positionieren eines Dichtelements abgewinkelt zu einem Arretierungselement, wobei das Arretierungselement mit dem Arbeitselement verbunden ist oder an diesem ausgeformt ist, Einrasten des Dichtelements in zumindest einen Rastabschnitt des Arretierungselements, Schwenken des Dichtelements in eine Ebene parallel zu dem Arretierungselement, bis eine dichtende Position erreicht wird, und Halten des Dichtelements in der dichtenden Position.

Die der vorliegenden Erfindung zugrundeliegende Erkenntnis besteht darin, dass eine Mehrfachverwendung nicht immer sinnvoll ist, wenn die Dichtelemente, beispielsweise bei Austausch der Instrumente, schnell und einfach getauscht werden müssen. Durch Umsetzung einer einfachen Bauart kann hingegen eine zur Einmalverwendung ausgelegte "Single-Use" Dichtung ausgebildet werden.

Die der vorliegenden Erfindung zugrundeliegende Idee besteht darin, das Dichtelement an einem Arretierungselement einzurasten, wobei durch einfache Handgriffe das Dichtelement von dem Arretierungselement entfernt oder an diesem fixiert werden kann.

Die Dichtungsvorrichtung kann zumindest teilweise umlaufend um einen Teil des Arbeitselements verlaufen, wobei der Teil in Richtung des Handgriffs ausgerichtet sein kann.

Bei dem zumindest einen Instrument kann es sich um eine Elektrode, Nadel, Laserfaser oder Ähnliches handeln. Pro Instrument kann eine Ausnehmung in der Dichtungsvorrichtung vorgesehen sein. Ebenso können pro Instrument zwei voneinander getrennte Ausnehmungen in der Dichtungsvorrichtung vorgesehen sein. Eine Abdichtung kann daher zumindest zwischen der jeweiligen Ausnehmung und dem jeweiligen Instrument stattfinden.

Darüber hinaus ist mit einer derartigen Dichtungsvorrichtung auch eine Abdichtung möglich, wenn noch kein Instrument in das Arbeitselement eingesetzt bzw. eingeschoben ist. Dies ermöglicht eine verbesserte Abdichtung in unterschiedlichen Zuständen, insbesondere mit oder ohne eingesetzten Instrumenten.

Ferner ist es auf diese Weise vorteilhaft möglich, das Dichtelement zur Einmalverwendung bzw. als "Single-Use" Dichtelement auszugestalten, und das Arretierungselement mehrfach zu verwenden.

Weiterhin kann mit dem Dichtelement sichergestellt werden, dass das Instrument leichtgängig in der Dichtung bewegt werden kann. Dazu kann die Dichtungsvorrichtung eine Weichkomponente aufweisen, insbesondere das Dichtelement.

Das Dichtelement kann insbesondere oberflächenbehandelt sein, beispielsweise durch Fluorieren, sodass das zumindest eine Instrument besonders voreilhaft und besonders leichtgängig geführt werden kann. So kann beispielsweise nur ein Instrument eingeführt werden. Des Weiteren können zwei, drei oder mehr als drei Instrumente jeweils separat eingeführt werden, wobei jedes der Instrumente in einer eigenen Ausnehmung geführt sein kann.

Das Arretierungselement kann unterschiedliche Ausgestaltungen aufweisen, beispielsweise kann es in oder an dem Arbeitselement ausgeformt sein. Dies bedeutet insbesondere, dass der Rastabschnitt direkt in oder an dem Arbeitselement ausgeformt ist. An dem Rastabschnitt können daher unterschiedliche Dichtelemente einfach und zeitsparend eingerastet bzw. entfernt werden.

Als Rastabschnitt ist beispielsweise eine Art Eingriff zu verstehen, in welchen das Dichtelement eingreifen kann. Der Rastabschnitt kann ein Hinterschnitt sein. Insbesondere kann der Rastabschnitt aus mehr als einem Eingriff oder mehr als einem Hinterschnitt ausgebildet sein.

Insbesondere kann der Rastabschnitt einen Randbereich des Dichtelements aufnehmen, um so das Dichtelement selbsttätig an dem Arretierungselement zu halten.

Selbsttätig bedeutet insbesondere, dass das Dichtelement ohne weitere Fixierungselemente und/oder Handgriffe an dem Arretierungselement gehalten werden kann. Dies kann durch Einrasten und/oder Klemmen in dem Rastabschnitt, insbesondere in einer Einhandbedienung, erfolgen.

Bei dem chirurgischen Instrument kann es sich um ein Resektoskop handeln. Eine Verwendung bei anderen chirurgischen Instrumenten ist ebenso denkbar.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer vorteilhaften Ausführungsform kann das Dichtelement auf einer Grundplatte angeordnet sein, wobei die Grundplatte einen zu dem Rastabschnitt korrespondierenden Rastabschnitt aufweist. Vorteilhaft umfasst dadurch die Dichtungsvorrichtung eine Hartkomponente und eine Weichkomponente. Die Grundplatte kann dabei als Hartkomponente ausgebildet sein. Das Dichtelement ist dementsprechend als Weichkomponente ausgebildet. Der Rastabschnitt an der Grundplatte kann beispielsweise als eine Nase ausgebildet sein, die in eine Vertiefung oder einen Hinterschnitt an dem Arretierungselement eingreifen und/oder einrasten kann.

Die Grundplatte kann größer als das Dichtelement ausgebildet sein, sodass beispielsweise an einer Seitenkante des Dichtelements der Rastabschnitt übersteht. Des Weiteren kann die Grundplatte an einer dem Rastabschnitt gegenüberliegenden Seite ebenso das Dichtelement überragen, sodass eine weitere Fixierungsmöglichkeit an einer Fläche der Grundplatte resultiert.

Gemäß einer Ausführungsform kann die Grundplatte einen Fixierungsabschnitt aufweisen, insbesondere einen Vorsprung, der in der dichtenden Position in eine Ausnehmung in dem Arretierungselement eingreift, um das Dichtelement in der durch die Rastabschnitte bestimmten, dichtenden Position an dem Arretierungselement dichtend zu fixieren. Der Fixierungsabschnitt kann in einem Bereich ausgebildet sein, in welchen die Grundplatte das Dichtelement überragt. Dadurch beeinflusst der Fixierungsabschnitt das Dichtelement nicht. Insbesondere ist der Fixierungsabschnitt beabstandet zu dem Dichtelement ausgebildet und nicht mit dem Dichtelement in Kontakt.

In einer weiteren Ausführungsform können unterschiedliche Dichtelemente an ein und derselben Grundplatte montiert werden. Dadurch kann eine einfache Herstellung von identischen Grundplatten erfolgen, auf welche unterschiedliche Dichtelement angeordnet werden können.

Gemäß einer vorteilhaften Ausführungsform kann der Rastabschnitt in einer Ebene mit der Grundplatte ausgeformt sein, und eine Dicke der Grundplatte nicht überragen. Dadurch kann die Grundplatte mit dem Dichtelement sehr nahe an dem Arretierungselement einrasten, und dadurch den Bereich zwischen dem Arbeitselement und dem zumindest einen Instrument abdichten.

Gemäß einer bevorzugten Ausführungsform kann der Fixierungsabschnitt seitlich an der Grundplatte, insbesondere hakenförmig, ausgeformt sein, und eine Dicke der Grundplatte überragen. Der Fixierungsabschnitt kann ein Vorsprung bzw. ein stiftförmiges Element sein, der/das an einem freien Ende eine wulstartige Verdickung aufweist. Dadurch kann beispielsweise ein Einrasten in dem Arretierungselement ermöglicht werden.

Gemäß einer besonders bevorzugten Ausführungsform können der Rastabschnitt und der Fixierungsabschnitt in unterschiedlichen Richtungen ausgerichtet sein, wobei der Rastabschnitt als Anschlag in eine erste Richtung und die Ausnehmung als Aufnahme und Arretierung in eine zweite Richtung dient.

Dadurch kann das Dichtelement besonders vorteilhaft an das Arretierungselement gepresst werden. Vorteilhafterweise ist der Rastabschnitt in dem Arretierungselement sowie die Ausnehmung in dem Arretierungselement derart angeordnet, dass das Dichtelement in dem eingerasteten Zustand in der dichtenden Position an dem Arretierungselement gehalten ist. Bevorzugt sind daher der Fixierungsabschnitt und das Dichtelement an derselben Oberfläche der Grundplatte angeordnet.

Gemäß einer vorteilhaften Ausführungsform kann die Grundplatte zwei Rastabschnitte aufweisen, die an einer Seitenkante der Grundplatte überstehen. Dadurch kann das Dichtelement besonders vorteilhaft gegen ein Verdrehen gesichert werden. Des Weiteren können die Rastabschnitte in einem Bereich oberhalb des zumindest einen Instruments an dem Arbeitselement angeordnet werden, wobei ein größerer Bereich zur Fixierung des Dichtelements ausgenutzt werden kann.

Gemäß einer Weiterbildung kann der Fixierungsabschnitt mittig zwischen den Rastabschnitten angeordnet sein. Insbesondere ist der Fixierungsabschnitt nicht in einer Linie mit den Rastabschnitten angeordnet, sondern parallel versetzt dazu. Beispielsweise können die Rastabschnitte in einem Randbereich (Seitenkante) an der Grundplatte angeordnet sein. Der Fixierungsabschnitt kann an einem dazu gegenüberliegenden Randbereich an der Grundplatte angeordnet sein. Das Dichtelement kann dazwischen angeordnet werden, wobei es weder mit den Rastabschnitten noch mit dem Fixierungsabschnitt kontaktiert sein kann.

Gemäß einer Ausführungsform kann das Dichtelement als Flachdichtung ausgebildet sein und eine im Wesentlichen ebene Form aufweisen. Dadurch wird für die gesamte Dichtungsvorrichtung besonders wenig Bauraum benötigt. Vorteilhafterweise ist das Dichtelement als Flachdichtung parallel zu einer Grundplatte angeordnet, wobei die Flachdichtung mit der Grundplatte eine geringe Dicke aufweist.

Bei einer vorteilhaften Ausführungsform kann das Dichtelement in der dichtenden Position auch bei nicht eingesetztem Instrument die Schnittstelle abdichtet und/oder das Dichtelement durch das Einsetzen des Instruments eine Perforierung erfahren. Das Dichtelement kann quasi mit dem zumindest einen Instruments durchstochen werden, sodass für jedes Instrument eine Öffnung entsteht, die genau an den Durchmesser jeden Instruments angepasst ist.

Bei einer vorteilhaften Ausführungsform kann die Grundplatte zur Aufnahme des zumindest einen Instruments zumindest eine Ausnehmung aufweisen, wobei das Dichtelement die zumindest eine Ausnehmung überspannt. Die Perforierung bei Einsetzen jedes Instruments liegt vorteilhafterweise direkt innerhalb des Bereichs, in welchen das Dichtelement die zumindest eine Ausnehmung überspannt. Dadurch kann jedes Instrument ebenso durch eine jeweilige Ausnehmung geführt werden. So können nebeneinander mehrere Instrumente eingesetzt werden, wobei jedes der Instrumente separat abgedichtet werden kann.

Bei einer vorteilhaften Ausführungsform kann das Arretierungselement zumindest eine Randung aufweisen, die um eine Öffnung zur Aufnahme des zumindest einen Instruments angeordnet ist. Die Randung kann beispielsweise in Form eines Wulstes oder einer Aufwölbung ausgebildet sein. Es können ebenso mehrere Randungen vorgesehen sein, die jeweils als umlaufende Aufwölbungen um ein jeweiliges Durchgangsloch zur Aufnahme zumindest einen Instruments ausgebildet sind. Durch die zumindest eine Randung kann die Dichtwirkung mit dem Arretierungselement optimiert werden. Die Randung kann in Richtung des Dichtelements ausgerichtet sein. Die Randungen können daher gegen das Dichtelement gepresst werden, wenn das Dichtelement an dem Arretierungselement einrastet. Das Dichtelement kann zu den Ausnehmungen korrespondierende Öffnungen aufweisen, um die Instrumente aufzunehmen.

Gemäß einer vorteilhaften Ausführungsform des Verfahrens kann das Dichtelement daher auch bei nicht eingesetztem Instrument die Schnittstelle abdichten und/oder das Dichtelement durch Einsetzen des zumindest einen Instruments zumindest eine Perforierung erfahren.

Gemäß einer vorteilhaften Ausführungsform des Verfahrens kann das Dichtelement mit einem zu dem Rastabschnitt korrespondierenden Rastabschnitt in das Arretierungselement eingreifen, und die Rastabschnitte eine Rotationsachse ausbilden, um welche das Dichtelement von der abgewinkelten Position bis zu der dichtenden Position rotiert wird. Auf diese Weise kann das Dichtelement besonders nahe an dem Arretierungselement angeordnet werden, wobei vorteilhafterweise eine Flachdichtung eingesetzt wird.

Gemäß einer vorteilhaften Ausführungsform des Verfahrens kann bei Erreichen der dichtenden Position das Dichtelement mit einem Fixierungsabschnitt an dem Arretierungselement fixiert werden, wobei der Fixierungsabschnitt als Vorsprung ausgebildet ist, der in der dichtenden Position in eine Ausnehmung in dem Arretierungselement eingreift. Der Rastabschnitt und die Ausnehmung in dem Arretierungselement sind in einem derartigen Abstand zueinander angeordnet, sodass das Dichtelement, insbesondere mit der Grundplatte, an dem Arretierungsabschnitt gehalten wird, sobald das Dichtelement in der dichtenden Position vorliegt. Ausgehend von der abgewinkelten Position rastet das Dichtelement daher erst an dem Arretierungselement, wenn ist die dichtende Position erreicht hat.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine isometrische Darstellung eines chirurgischen Instruments mit einer Dichtungsvorrichtung;
- Fig. 2: eine Dichtungsvorrichtung an einem chirurgischen Instrument während der Montage;
- Fig. 3: eine Dichtungsvorrichtung in drei Ansichten;
- Fig. 4: eine weitere isometrische Darstellung eines chirurgischen Instruments mit einer Dichtungsvorrichtung;
- Fig. 5: ein Arretierungselement in einer möglichen Ausführungsform.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

### BESCHREIBUNG VON AUSFÜHRUNGSBEISPIELEN

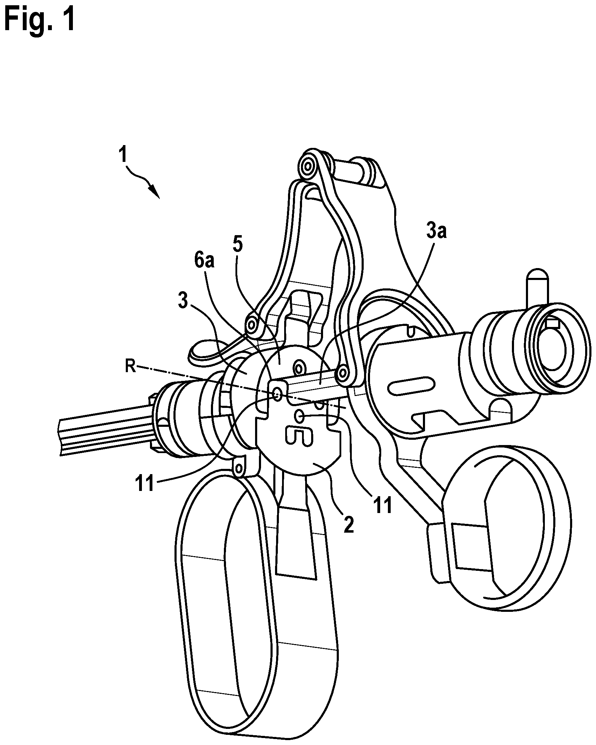

Fig. 1 zeigt eine isometrische Darstellung eines chirurgischen Instruments mit einer Dichtungsvorrichtung 1.

Ein Dichtelement 2 ist zur Abdichtung einer Schnittstelle zwischen einem Arbeitselement 3 und zumindest einem Instrument 4 (nicht dargestellt) ausgebildet. Dazu ist ein Arretierungselement 5 vorgesehen, das mit dem Arbeitselement 3 verbindbar ist oder an diesem ausgeformt ist. Das Arretierungselement 5 kann beispielsweise an dem Arbeitselement 3 eingerastet, angeklebt oder anderweitig befestigt sein. Ebenso kann das Arretierungselement 5 einteilig mit dem Arbeitselement 3 geformt sein.

Das Arretierungselement 5 dient zur Fixierung des Dichtelements 2 und weist zumindest einen Rastabschnitt 6a auf. Der Rastabschnitt 6a ist auf einer Rotationsachse R angeordnet, um welche das Dichtelement 2 rotieren kann. Dadurch kann das Dichtelement 2 einfach und schnell montiert bzw. demontiert werden. Das Dichtelement kann dabei quasi von unten in den Rastabschnitt 6a eingeführt werden, und so an dem Arbeitselement 3 montiert werden. Insbesondere ist die Dichtungsvorrichtung 1, bzw. das Dichtelement 2, zur Einmalverwendung, demnach als Single-use Dichtung, ausgebildet und dient beispielsweise zur Anwendung bei einem Resektoskop oder generell für medizinische Instrumente.

In der Darstellung in Fig. 1 ist das chirurgische Instrument zur Aufnahme von zwei Instrumenten 4 ausgebildet, wobei das Dichtelement 2 drei Ausnehmungen 11 aufweist (zwei sind erkennbar, eine ist verdeckt). Die Ausnehmungen können erst bei Durchstechen mit dem jeweiligen Instrument 4 in dem Dichtelement 2 entstehen, sodass eine Abdichtung bei nicht eingesetztem Instrument 4 vorliegt. In der Grundplatte 7 sind die Ausnehmungen 11 vorteilhafterweise bereits vorhanden. Auf dem Dichtelement 5 kann daher zumindest eine Markierung bzw. Perforierung vorgesehen sein, um die Position der Ausnehmung 11 in der Grundplatte 7 zu kennzeichnen, sodass das Instrument 4 an entsprechender Stelle eingesetzt und das Dichtelement 5 durchstechen kann.

Die Ausnehmungen 11 können zumindest teilweise umlaufend um einen Teil 3a des Arbeitselements 3 an der Dichtvorrichtung 1 angeordnet sein.

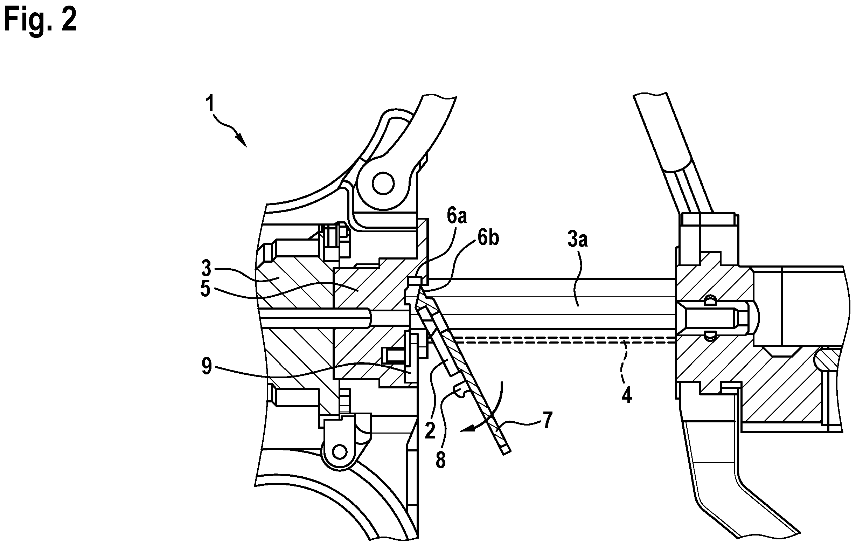

In Fig. 2 ist eine Dichtungsvorrichtung 1 an einem chirurgischen Instrument während der Montage gezeigt.

Für die Montage bzw. das Verfahren zur Montage erfolgt zunächst ein Positionieren des Dichtelements 2 abgewinkelt zu dem Arretierungselement 5, wie in der Fig. 2 dargestellt. Das Dichtelement 2 wird in zumindest einen Rastabschnitt 6a des Arretierungselements 5 eingerastet, wobei das Dichtelement 2 in dieser Ausführungsform eine Grundplatte 7 mit einem Rastabschnitt 6b aufweist. Anschließend wird das Dichtelement 2 derart verschwenkt, dass es in einer Ebene parallel zu dem Arretierungselement 5 angeordnet ist, und in dieser Position eine dichtende Position erreicht. Parallel bedeutet insbesondere, dass das Dichtelement 2 vollflächig mit einer Oberfläche des Arretierungselements 5 kontaktiert ist. Zur Fixierung ist an dem Dichtelement 2, bzw. an der Grundplatte 7, ein Fixierungsabschnitt 8 vorgesehen, der an einer Oberfläche der Grundplatte 7 übersteht, und damit die Dicke der Grundplatte 7 überragt. Der Fixierungsabschnitt 8 und der zumindest eine Rastabschnitt 6b sind in unterschiedlichen Richtungen ausgerichtet, sodass eine sichere Fixierung des Dichtelements 2 an dem Arretierungselement 5 erfolgen kann. Der Fixierungsabschnitt 8 kann beispielsweise hakenförmig oder nasenförmig ausgebildet sein, wobei an einem freien Ende des Fixierungsabschnitts 8 eine wulstartige Verdickung vorgesehen sein kann. Diese wulstartige Verdickung kann beispielsweise die Hakenform ausbilden. Dadurch kann der Fixierungsabschnitt 8 in einer Ausnehmung 9 in dem Arretierungselement 5 einrasten, und das Dichtelement 2 in der abgedichteten Position fixieren.

Ein Instrument 4 ist mit gestrichelten Linien angedeutet. Dieses kann anschließend eingeschoben werden, wodurch das Dichtelement 2 perforiert werden kann.

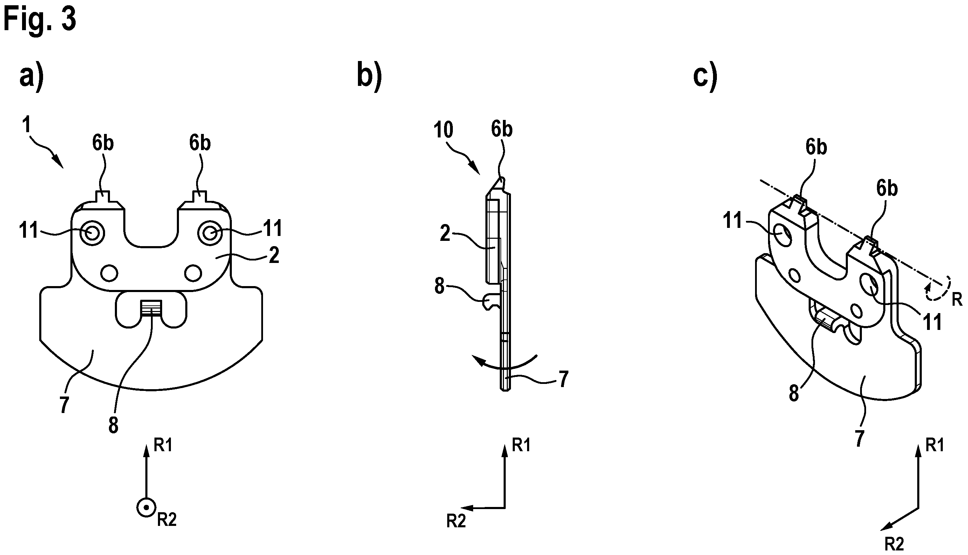

Fig. 3 zeigt eine Dichtungsvorrichtung 1 in drei Ansichten.

Die Grundplatte 7 weist zwei Rastabschnitte 6b auf, die an einer Seitenkante 10 der Grundplatte 7 überstehen. Unterhalb der Rastabschnitte 6b können mehrere Ausnehmungen 11 vorgesehen sein, durch welche jeweils ein Instrument 4 (nicht dargestellt) hindurchgeführt werden kann. Beim Hindurchführen des zumindest einen Instruments 4 kann das Dichtelement 2, welches die Ausnehmung 11 überspannt, durchstochen werden, sodass eine optimale Abdichtung um jedes Instrument 4 erreicht werden kann. Jedes Instrument kann daher separat abgedichtet werden.

Die Darstellung zeigt bereits aus Gründen der Übersichtlichkeit bereits zwei Ausnehmungen 11, wobei diese im nicht durchstochenen Zustand nur angedeutet erkennbar sein können bzw. nicht erkennbar sein können. Die Anordnung der Ausnehmungen 11 kann von den dargestellten Positionen abweichend ausgestaltet sein.

Vorteilhaft kann die Dichtungsvorrichtung 1 auch vor Einsetzen des zumindest einen Instruments 4 eine dichtende Funktion aufweisen. Ein leichtgängiger Lauf des Instruments 4 kann beispielsweise durch eine Oberflächenbehandlung des Dichtelements 2 erreicht werden. Das Dichtelement 2 ist bevorzugt als Weichkomponente ausgebildet, wobei eine Oberflächenbehandlung beispielsweise durch Florieren erfolgen kann. Die Grundplatte 7 ist bevorzugt als Hartkomponente ausgeformt, und ist größer als das Dichtelement 2 ausgebildet. Demnach überragt die Grundplatte 7 das Dichtelement 2. An einer gegenüberliegenden Seite zu der Seitenkante 10 kann an der Grundplatte 7 der Fixierungsabschnitt 8 angeordnet sein. Der Fixierungsabschnitt 8 ist bevorzugt in die Raumrichtungen R2 ausgerichtet, während die Rastabschnitt der 6b in die Raumrichtungen R1 ausgerichtet sind. Die Raumrichtungen R1, R2 sind bevorzugt quer zueinander ausgerichtet, insbesondere orthogonal. Dadurch kann das Dichtelement 2 in zwei unterschiedlichen Raumrichtungen an dem Arretierungselement 5 fixiert werden. Während der Montage erfolgt eine Rotation um die Rotationsachse R, und zwar so lange, bis der Fixierungsabschnitt 8 an dem Arretierungselement 5 eingerastet ist. Wenn der Fixierungsabschnitt 8 in das Arretierungselement 5 eingerastet ist, ist bevorzugt das Dichtelement 2 vollflächig mit dem Arretierungselement 5 in Kontakt und damit parallel zu dem Arretierungselement 5 ausgerichtet. Insbesondere wird in diesem Zustand das Dichtelement 2 an das Arretierungselement 5 gepresst, und zwar durch die Fixierung durch die Rastabschnitte 6a, 6b und durch den Fixierungsabschnitt 8.

Mit anderen Worten kann eine Fixierung des Dichtelements 2 an dem Arretierungselement 5 durch ein Halteelement, in Form des zumindest einen Rastabschnitts 6a, 6b, und durch zumindest einen Schnapphaken, in Form des Fixierungsabschnitts 8, erfolgen. Somit erfolgen eine Positionierung und eine Fixierung.

In der dargestellten Ausführungsform ist das Dichtelement 2 als Flachdichtung ausgebildet, sodass es nur wenig Bauraum benötigt. Es kann zudem einfach montiert und demontiert werden, und durch die unterschiedlichen Rast- bzw. Fixierungsabschnitte sicher in der Position zwischen dem Arbeitselement 3 und dem Instrument 4 gehalten werden.

Der Fixierungsabschnitt 8 ist bevorzugt an der Oberfläche an der Grundplatte 7 vorgesehen, an welche auch das Dichtelement 2 angeordnet ist. Dadurch kann ebenso Bauraum eingespart werden, und weiteren kann das Dichtelement 2 an dem Arretierungselement 5 sicher und dichtend fixiert werden.

Weiterhin kann ein leichtgängiger Instrumentenlauf erfolgen, da jede Öffnung in dem Dichtelement 2 exakt an die Größe des Instruments 4 angepasst werden kann. Die Dichtungsvorrichtung 1 eignet sich durch die einfache Bauart und die wenigen Komponenten zur Einmalverwendung und kann dadurch wirtschaftlich umgesetzt werden.

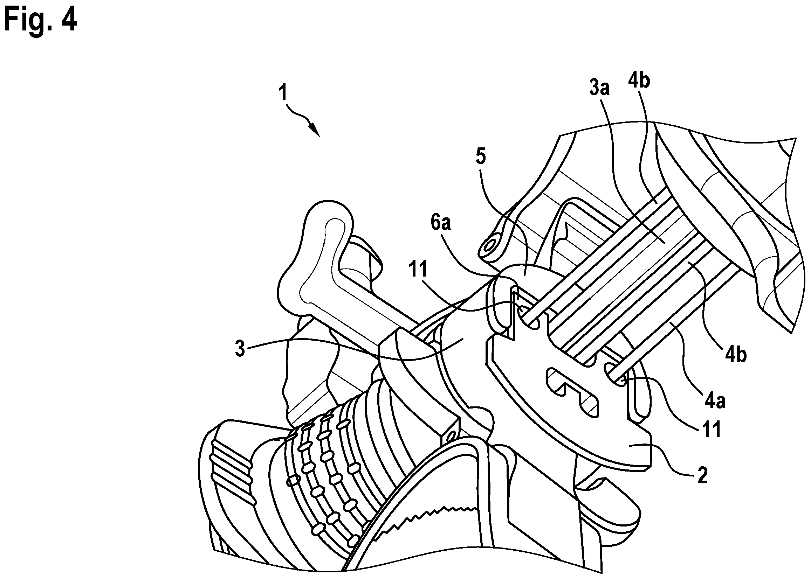

Fig. 4 zeigt eine weitere isometrische Darstellung eines chirurgischen Instruments mit einer Dichtungsvorrichtung 1. Hierbei sind zwei eingesetzte Instrumente 4a, 4b erkennbar. Bei jedem der Instrumente 4a, 4b kann es sich um Elektroden, Nadeln, Laserfaser oder ähnliches handeln. Diese können um einen Teil 3a des Arbeitselements 3 angeordnet werden.

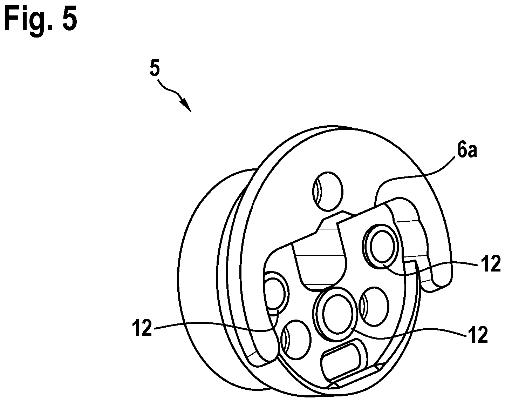

Fig. 5 zeigt ein Arretierungselement 5 in einer möglichen Ausführungsform. Diese kann zumindest eine Randung 12 oder mehrere Randungen 12, beispielsweise in Form von Wülsten, aufweisen, um die Dichtwirkung mit dem Arretierungselement 5 zu optimieren. Die Randungen 12 können in Richtung des Dichtelements 2 ausgerichtet sein. Das Dichtelement 5 weist zu den Ausnehmungen 11 korrespondierende Öffnungen auf, um die Instrumente 4 aufzunehmen.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

Beispielsweise kann die Position der Rastabschnitte 6b bzw. der Rastabschnitte 6a unterschiedliche Ausgestaltungen aufweisen.

Des Weiteren kann der Fixierungsabschnitt 8 an einer anderen Position an der Grundplatte 7 vorgesehen sein.

Ebenso können zwei Fixierungsabschnitte 8 an der Grundplatte 7, insbesondere an einer Oberfläche der Grundpatte 7, angeordnet werden, um für unterschiedliche Montagesituationen die Dichtungsvorrichtung 1 anzupassen.

### Bezugszeichenliste

- 1: Dichtungsvorrichtung
- 2: Dichtelement
- 3: Arbeitselement
- 3a: Teil des Arbeitselements
- 4: Instrument
- 5: Arretierungselement
- 6a: Rastabschnitt
- 6b: Rastabschnitt
- 7: Grundplatte
- 8: Fixierungsabschnitt
- 9: Ausnehmung
- 10: Seitenkante
- 11: Ausnehmung
- 12: Randung

## Patentansprüche

1. Dichtungsvorrichtung (1) für zumindest ein chirurgisches Instrument, mit: einem Dichtelement (2), das zur Abdichtung einer Schnittstelle zwischen einem Arbeitselement (3) und zumindest einem Instrument (4) ausgebildet ist, einem Arretierungselement (5), das mit dem Arbeitselement (3) verbindbar ist oder an diesem ausgeformt ist, wobei das Arretierungselement (5) zur Fixierung des Dichtelements (2) ausgebildet ist und zumindest einen Rastabschnitt (6a) aufweist, wobei das Dichtelement (2) zum Eingreifen in den Rastabschnitt (6a) und selbsttätigen Halten an dem Arretierungselement (5) in einer die Schnittstelle abdichtenden Position ausgebildet ist.

2. Dichtungsvorrichtung (1) nach Anspruch 1, **dadurch gekennzeichnet, dass** das Dichtelement (2) auf einer Grundplatte (7) angeordnet ist, wobei die Grundplatte (7) einen zu dem Rastabschnitt (6a) korrespondierenden Rastabschnitt (6b) aufweist.

3. Dichtungsvorrichtung (1) nach Anspruch 2, **dadurch gekennzeichnet, dass** die Grundplatte (7) einen Fixierungsabschnitt (8) aufweist, insbesondere einen Vorsprung, der in der dichtenden Position in eine Ausnehmung (9) in dem Arretierungselement (5) eingreift, um das Dichtelement (2) in der durch die Rastabschnitte (6a, 6b) bestimmten, dichtenden Position an dem Arretierungselement (5) dichtend zu fixieren.

4. Dichtungsvorrichtung (1) nach Anspruch 2 oder 3, **dadurch gekennzeichnet, dass** der Rastabschnitt (6b) in einer Ebene mit der Grundplatte (7) ausgeformt ist, und eine Dicke der Grundplatte nicht überragt.

5. Dichtungsvorrichtung (1) nach einem der Ansprüche 3 oder 4, **dadurch gekennzeichnet, dass** der Fixierungsabschnitt (8) seitlich an der Grundplatte (7), insbesondere hakenförmig, ausgeformt ist, und eine Dicke der Grundplatte (7) überragt.

6. Dichtungsvorrichtung (1) nach einem der Ansprüche 3 bis 5, **dadurch gekennzeichnet, dass** der Rastabschnitt (6b) und der Fixierungsabschnitt (8) in unterschiedlichen Richtungen ausgerichtet sind, wobei der Rastabschnitt (6a) als Anschlag in eine erste Richtung (R1) und die Ausnehmung (9) als Aufnahme und Arretierung in eine zweite Richtung (R2) dient.

7. Dichtungsvorrichtung (1) nach einem der Ansprüche 2 bis 6, **dadurch gekennzeichnet, dass** die Grundplatte (7) zwei Rastabschnitte (6b) aufweist, die an einer Seitenkante (10) der Grundplatte (7) überstehen.

8. Dichtungsvorrichtung (1) nach Anspruch 7, **dadurch gekennzeichnet, dass** der Fixierungsabschnitt (8) mittig zwischen den Rastabschnitten (6b) angeordnet ist.

9. Dichtungsvorrichtung (1) nach einem der vorstehenden Ansprüche, **dadurch gekennzeichnet, dass** das Dichtelement (2) als Flachdichtung ausgebildet ist und eine im Wesentlichen ebene Form aufweist.

10. Dichtungsvorrichtung (1) nach einem der vorstehenden Ansprüche, **dadurch gekennzeichnet, dass** das Dichtelement (2) in der dichtenden Position auch bei nicht eingesetztem Instrument (4) die Schnittstelle abdichtet und/oder das Dichtelement (2) durch das Einsetzen des zumindest einen Instruments (4) zumindest eine Perforierung erfährt.

11. Dichtungsvorrichtung (1) nach einem der Ansprüche 2 bis 10, **dadurch gekennzeichnet, dass** die Grundplatte (2) zur Aufnahme des zumindest einen Instruments (4) zumindest eine Ausnehmung (11) aufweist, wobei das Dichtelement (2) die zumindest eine Ausnehmung (11) überspannt.

12. Dichtungsvorrichtung (1) nach einem der vorstehenden Ansprüche, **dadurch gekennzeichnet, dass** das Arretierungselement (5) zumindest eine Randung (12) aufweist, die um eine Öffnung zur Aufnahme des zumindest einen Instruments (4) angeordnet ist.

13. Verfahren zur Montage einer Dichtungsvorrichtung (1) zur Abdichtung einer Schnittstelle zwischen einem Arbeitselement (3) und zumindest einem Instrument (4) eines chirurgischen Instruments, insbesondere mit einer Dichtungsvorrichtung (1) nach einem der vorstehenden Ansprüche, mit den Schritten:
Positionieren eines Dichtelements (2) abgewinkelt zu einem Arretierungselement (5), wobei das Arretierungselement (5) mit dem Arbeitselement (3) verbunden ist oder an diesem ausgeformt ist, Einrasten des Dichtelements (2) in zumindest einen Rastabschnitt (6a) des Arretierungselements (5), Schwenken des Dichtelements (2) in eine Ebene parallel zu dem Arretierungselement (5), bis eine dichtende Position erreicht wird, und Halten des Dichtelements (2) in der dichtenden Position.

14. Verfahren zur Montage nach Anspruch 13, **dadurch gekennzeichnet, dass** das Dichtelement (2) auch bei nicht eingesetztem Instrument (4) die Schnittstelle abdichtet und/oder das Dichtelement (2) durch Einsetzen des zumindest einen Instruments (4) zumindest eine Perforierung erfährt.

15. Verfahren zur Montage nach Anspruch 13 oder 14,
**dadurch gekennzeichnet, dass** das Dichtelement (2) mit einem zu dem Rastabschnitt (6a) korrespondierenden Rastabschnitt (6b) in das Arretierungselement (5) eingreift, und die Rastabschnitte (6a, 6b) eine Rotationsachse (R) ausbilden, um welche das Dichtelement (2) von der abgewinkelten Position bis zu der dichtenden Position rotiert wird.

16. Verfahren zur Montage nach einem der Ansprüche 13 bis 15, **dadurch gekennzeichnet, dass** bei Erreichen der dichtenden Position das Dichtelement (2) mit einem Fixierungsabschnitt (8) an dem Arretierungselement (5) fixiert wird, wobei der Fixierungsabschnitt (8) als Vorsprung ausgebildet ist, der in der dichtenden Position in eine Ausnehmung (9) in dem Arretierungselement (5) eingreift.
